# EUROPÄISCHE PATENTSCHRIFT

(11) **EP 0 701 992 B1**
(45) Veröffentlichungstag und Bekanntmachung des Hinweises auf die Patenterteilung: **17.11.1999**
(21) Anmeldenummer: 95113674.6
(22) Anmeldetag: 31.08.1995
(51) Int. Cl.: C07C 209/02, C07D 239/42, C07D 213/74

(54) **Verfahren zur Herstellung nitrosubstituierter Arylamine**
Method of preparation of nitro substituted arylamines
Procédé pour la préparation des arylamines substituées par les groupes nitro

(30) Priorität: 13.09.1994 DE 44432556 F
(43) Veröffentlichungstag der Anmeldung: 20.03.1996
(73) Patentinhaber: BAYER AG, 51368 Leverkusen (DE)
(72) Erfinder: Jautelat, Manfred, Dr., D-51399 Burscheid (DE)

(56) Entgegenhaltungen:
- EP-A- 0 566 783
- US-A- 5 117 063

## Beschreibung

Die Erfindung betrifft ein Verfahren zur Herstellung von nitrosubstituierten Arylaminen aus Nitroaromaten, Aminen und Sauerstoff in Gegenwart von Basen.

Aromatische Amine sind wichtige Zwischenprodukte für die Herstellung von Farbstoffen, Pflanzenschutzmittel, Pharmazeutika und für die Fotoindustrie.

Technisch bedeutsame Verfahren zur Herstellung von aromatischen Aminen sind die Reduktion von Nitrogruppen in leicht zugänglichen Nitroaromaten oder die Umsetzung von Halogenaromaten mit Ammoniak oder Aminen. Obwohl diese Verfahren im großen Umfange technisch genutzt werden, ergeben sich wegen der mehrstufigen Reaktion oft nicht zufriedenstellende Ausbeuten. Auch die direkte Aminierung von Nitrobenzol mit Acetanilid in Gegenwart von Basen in DMSO ist bekannt, wobei als Hauptprodukt p-Nitrosodiphenylamin entsteht (Tetrahedron Lett. 1990, 22, 3217-3220). Ferner ist ein Verfahren zur Herstellung von N-aliphatischsubstituierten p-Phenylen-diaminen beschrieben, durch Umsetzung von Nitrobenzol mit aliphatischen Aminen in Gegenwart von Base und Protonen-haltigen Substanzen (US-Patent 5.252.737). Desweiteren ist ein Verfahren zur Herstellung von p-nitroaromatischen Amiden und Aminen bekannt, das die Umsetzung von Nitrobenzol mit Amiden bzw. Aminen ohne Oxidationsmittel in Gegenwart von speziellen Basen, wie Tetraalkylammoniumhydroxiden, in Gegenwart von Protonen-haltigen Stoffen beschreibt (WO 93/24447, US-Pat. 5.117.063 und J. Am. Chem. Soc. 1992, 114, 9237). Diese Verfahren erfordern spezielle Bedingungen oder Basen und/oder liefern Ausbeuten, die nicht immer zufriedenstellend sind.

Überraschenderweise wurde nun ein allgemein anwendbares Verfahren zur direkten Aminierung von nitrosubstituierten Aromaten mit Aminen in Gegenwart von einfachen Basen und Sauerstoff gefunden. Die Umsetzungen führen in guten Ausbeuten zu den entsprechenden Aminen.

Gegenstand der Erfindung ist ein Verfahren zur Herstellung aromatischer Amine der Formel 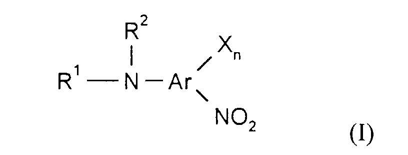 worin
- Ar: einen mono- oder polycyclischen, vorzugsweise mono- oder bicyclischen, aromatischen Rest mit 4 bis 16 C-Atomen, der auch 1 bis 2 Heteroatome aus der Reihe bestehend aus N, O und S enthalten kann,
- R¹: einen mono- oder polycyclischen, vorzugsweise mono- oder bicyclischen, aromatischen Rest mit 5 bis 16 Ringgliedern, die aus Kohlenstoffatomen und gegebenenfalls bis zu 3 Heteroatomen aus der Reihe N, O und S bestehen, ausgewählt aus der Gruppe Pyrazol, Imidazol, 1,2,4-Triazol, Thiazol, Benzol, Naphthalin, Pyridin, Chinolin, Pyrimidin,
- R²: Wasserstoff, C₁-C₈-Alkyl, C₂-C₈-Alkenyl, C₃-C₇-Cycloalkyl, C₆-C₁₄-Aryl, wobei diese Substituenten 1- bis 3-fach durch Halogen, C₁-C₄-Alkyl, und/oder C₁-C₄-Alkoxy substituiert sein können,
- X: Halogen, Cyano, C₁-C₄-Alkyl, halogeniertes C₁-C₄-Alkyl, C₁-C₄-Alkoxy, halogeniertes C₁-C₄-Alkoxy, Trifluormethylmercapto, C₁-C₄-Alkylsulfonyl, Carboxyl oder Nitro,
- n: Null, 1, 2 oder 3, vorzugsweise Null, 1 oder 2, bedeuten,
wobei bei n > 1 die Substituenten X verschieden sein können,
wonach man Nitroaromaten der Formel 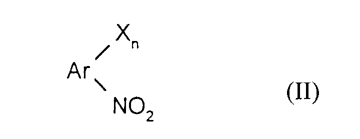 worin X, Ar und n die oben angegebenen Bedeutungen haben,
mit Aminen der Formel 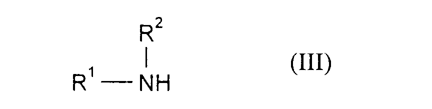 worin R¹ und R² die oben angegebenen Bedeutungen besitzen,
in Gegenwart von Basen unter Einleiten von Sauerstoff in polaren Lösungsmitteln umsetzt.

Bevorzugte aromatische C₄-C₁₆-Reste Ar umfassen beispielsweise Benzol-, Naphthalin-, Pyridin-, Chinolin- und Thiophenreste, vorzugsweise Benzol- und Naphthalinreste.
"C₁-C₈-Alkyl" und "C₁-C₄-Alkyl" umfassen lineare und verzweigte Reste wie Methyl, Ethyl, Isopropyl und n-, sek- und tert-Butyl.
"C₂-C₈-Alkenyl" umfaßt Vinyl und Allyl.
"C₃-C₇-Cycloalkyl" umfaßt Cyclopropyl, Cyclopentyl und Cyclohexyl.
"C₆-C₁₄-Aryl" steht für unsubstituierte oder substituierte Arylreste wie Phenyl oder Naphthyl, die einfach oder mehrfach durch Halogen, C₁-C₆-Alkyl, Nitro, Amino,
C₁-C₆-Alkoxy, C₁-C₆-Alkylthio, Sulfosäure, Hydroxy, Formyl, Benzoyl, Carboxyl, Cyano, Phenyl und Phenyl-C₁-C₆-alkyl substituiert sein können.
"Halogen" steht für Brom, Iod, vorzugsweise Fluor und Chlor.
"Halogeniertes C₁-C₄-Alkyl" umfaßt z. B. Trifluormethyl und Dichlorfluormethyl.
"C₁-C₄-Alkoxy" bedeutet vorzugsweise Methoxy; "halogeniertes C₁-C₄-Alkoxy" steht vorzugsweise für Trifluormethoxy.
"C₁-C₄-Alkylsulfonyl" steht vorzugsweise für Methylsulfonyl.

Bevorzugte Nitroaromaten (II) umfassen beispielsweise Nitrobenzol, m-Chlornitrobenzol, m-Nitrobenzonitril, m-Trifluormethylnitrobenzol, 3-Fluor-nitrobenzol, 3-Nitrotoluol, 3-Trifluormethoxynitrobenzol, 3-Trifluormethylthio-nitrobenzol, 3,5-Dichlornitrobenzol, 2-Nitrobenzonitril, 2-Nitrobenzoesäure, 1-Nitronaphthalin, 2-Nitronaphthalin, 2-Nitrothiophen, 3-Nitrothiophen, 2-Nitrofuran, N-alkylierte und N-arylierte 2- und 3-Nitropyrrole, 2-, 3- und 4-Nitro-pyridin, 4-Ethoxy-3-nitropyridin, 5-, 6- und 8-Nitrochinolin.

Bevorzugte Amine der Formel (III) umfassen beispielsweise Anilin, N-Methylanilin, N-Butylanilin, N-Isobutylanilin, Diphenylamin, 2-Chloranilin, 2,4-Dichloranilin, 2,6-Dichloranilin, 2,4,6-Trichloranilin, 3-Nitroanilin, 4-Nitroanilin, N-Cyclohexyl-4-nitroanilin, 4-Chlor-2-nitroanilin, 2,6-Dichlor-4-nitroanilin, 2-Brom-4-nitroanilin, 3-Methylanilin, 4-Chlor-2-trifluormethylanilin, 3-Trifluormethylanilin, N-Butyl-3-methylanilin, 3,5-Dimethylanilin, 3,5-Bis-trifluormethyl-anilin, 4-Aminobiphenyl, 1-Naphthylamin, 1-Aminopyren, 3-Amino-dibenzofuran, 2-Aminopyridin, 2-Amino-4,6-dimethylpyrimidin, 2-Amino-4-methoxy-6-methylpyrimidin, 2-Amino-4,6-dimethylpyrimidin, 1,3,5-triazin, 1-Amino-1,3,4-triazol, 2-Aminothiazol, 5-Amino-3-phenyl-1,2,4-thiadiazol.

Geeignete Basen sind sowohl organische wie anorganische Basen; bevorzugt werden anorganischen Basen, wie z. B. Alkalihydroxide, Alkaliamide, Alkalialkoxide oder Alkalihydride. Besonders bevorzugt sind Alkalihydroxide, wie z. B.

Lithiumhydroxid, Natriumhydroxid, Kaliumhydroxid, Caesiumhydroxid, Kaliumtert.-butoxid. Vorzugsweise setzt man die Basen in Form von Pulvern oder Mikrogranulat (Mikropills) ein.

Sauerstoff kann als reines Gas oder besonders bevorzugt in Gemischen mit anderen Gasen, beispielsweise in Form von Luft, eingesetzt werden.

Geeignete Lösungsmittel für die Herstellung der erfindungsgemäßen Verbindungen (I) umfassen organische und anorganische Lösungsmittel. Bevorzugte organische Lösungsmittel sind polare aprotische, wie beispielsweise Dimethylsulfoxid, Dimethylformamid, N-Methylpyrrolidon, Pyridin, Dioxan, THF, Acetonitril, Sulfolan, Dimethylsulfon und deren Gemische. Ein bevorzugtes anorganisches Lösungsmittel ist beispielsweise flüssiger Ammoniak. Geringe Mengen, d. h. bis zu 10 Gew.-%, bezogen auf gesamtes Lösungsmittel, an Protonen-haltigen Lösungsmitteln, wie etwa Wasser, sind akzeptabel. Das bevorzugte Lösungsmittel ist Dimethylsulfoxid.

Die Reaktion kann innerhalb eines weiten Temperaturbereichs durchgeführt werden. Im allgemeinen arbeitet man bei Temperaturen zwischen -35°C und 120°C, vorzugsweise zwischen 20°C und 100°C, insbesondere zwischen 20 und 80°C.

Bei der Durchführung des erfindungsgemäßen Verfahrens arbeitet man im allgemeinen unter Normaldruck; es ist aber auch möglich, unter erhöhtem oder vermindertem Druck zu arbeiten.

Bei der Durchführung des erfindungsgemäßen Verfahrens setzt man auf 1 mol des Nitroaromaten der Formel (II) im allgemeinen 0,5 bis 10 mol , vorzugsweise 0,7 bis 2 mol an Aminen der Formel (III) sowie 1 bis 10 Äquivalente, vorzugsweise 2 bis 6 Äquivalente Base ein. Sauerstoff wird vorzugsweise unverdünnt oder verdünnt im Überschuß eingeleitet.

Verwendet man 3-Nitrobenzotrifluorid, Anilin und Luft als Ausgangssstoffe und Natriumhydroxid als Base, so kann der Verlauf des Verfahrens zur Herstellung von nitrosubstituierten Aminen durch das folgende Formelschema wiedergegeben werden:

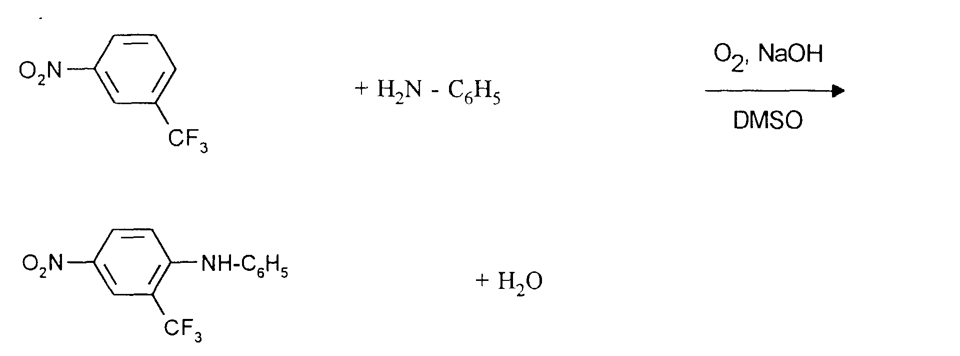

Die Ausgangsstoffe der Formeln (II) und (III) sind bekannt oder können nach bekannten Verfahren hergestellt werden.

Die Aufarbeitung kann nach den üblichen Methoden erfolgen. Im allgemeinen verfährt man in der Weise, daß man das Reaktionsgemisch mit Wasser stark verdünnt und das sich abscheidende Reaktionsprodukt abtrennt und isoliert, oder man verdünnt mit Wasser und extrahiert mit einem wenig mit Wasser mischbaren organischen Lösungsmittel. Aus der organischen Phase isoliert man das Produkt, nachdem diese getrocknet und eingeengt wurde.

### Beispiele

### Beispiel 1

### 2-(4-Nitro-2-trifluormethylphenyl-amino)-pyridin

1,91 g (10 mmol) 3-Nitrobenzotrifluorid, 1,88 g (20 mmol) 2-Aminopyridin und 1,2 g (30 mmol) Natriumhydroxid in Form von Mikroperlen werden in 30 ml abs. DMSO unter Durchleiten von trockener Luft 6 h auf 50°C erhitzt. Nach dem Abkühlen wird mit Essigsäureethylester verdünnt und mit Wasser mehrfach ausgeschüttelt. Nach Trocknen und Abziehen des organischen Lösungsmittels erhält man 3,8 g Rohprodukt, das durch Chromatographie über Kieselgel mit Petrolether und Ethylacetat (Volumenverhältnis 1 : 1) gereinigt wird. So gewinnt man 2,3 g (8,1 mmol, 81 %) 2-(4-Nitro-2-trifluormethylphenyl-amino)-pyridin vom Fp. 83 - 84°C.

### Beispiel 2

### 1 -(4-Chlor-2-trifluormethylphenyl)-amino-4-nitro-2-trifluormethylbenzol

1,91 g (10 mmol) 3-Nitrobenzotrifluorid, 1,96 g (10 mmol) 4-Chlor-2-trifluormethylanilin und 1,2 g (30 mmol) NaOH-Mikropills werden in 30 ml abs. DMSO unter Überleiten von trockener Luft 24 h auf 50°C erhitzt. Die Aufarbeitung mit Ethylacetat und gesättigter, wäßriger Natriumcarbonatlösung führt zu 3,8 g (10 mmol, 100 %) obigem Produkt, das nach chromatographischer Reinigung über Kieselgel mit Petrolether/Ethylacetat (Volumenverhältnis 10 : 1) einen Fp. von 84 - 85°C aufweist.

### Beispiel 3

### N-Methyl-N-(4-nitro-2-trifluormethylphenyl)-anilin

Zu 40 ml flüssigem Ammoniak werden bei -40°C 1,91 g (10 mmol) 3-Nitrobenzotrifluorid, 1,09 g (10 mmol) N-Methylanilin und 1,2 g (30 mmol) NaOH-Mikropills zugegeben. Dann wird getrocknete Luft durchgeleitet und die Mischung unter NH₃-Rückfluß 4 h gerührt. Nach dem Abdampfen von Ammoniak wird der Rückstand mit gesättigter, wäßriger Sodalösung verdünnt und mit Ethylacetat mehrfach ausgeschüttelt. Aus der organischen Phase bleiben nach Trocknen und Abziehen des Lösemittels 3,0 g Rohprodukt zurück. Durch Chromatographie über Kieselgel mit Petrolether/Ethylacetat (Volumenverhältnis 9 : 1) werden 1,2 g (4,1 mmol, 41 %) N-Methyl-N-(4-nitro-2-trifluormethylphenyl)-anilin isoliert.

Entsprechend den Beispielen 1 bis 3 werden folgende Verbindungen hergestellt:

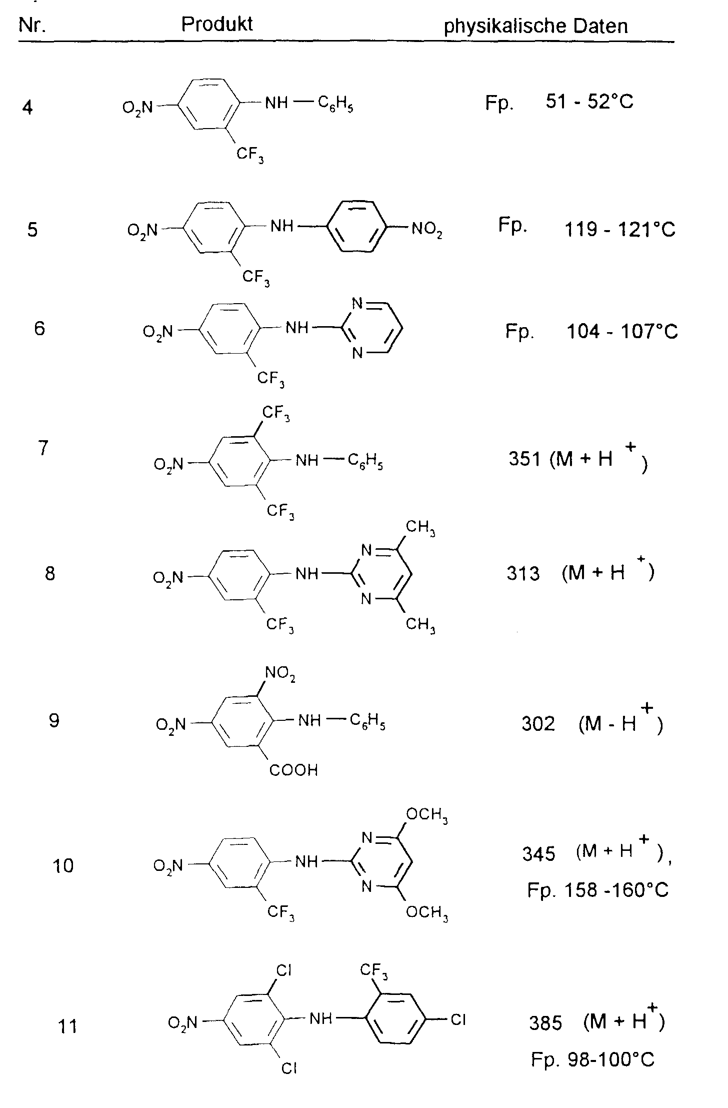

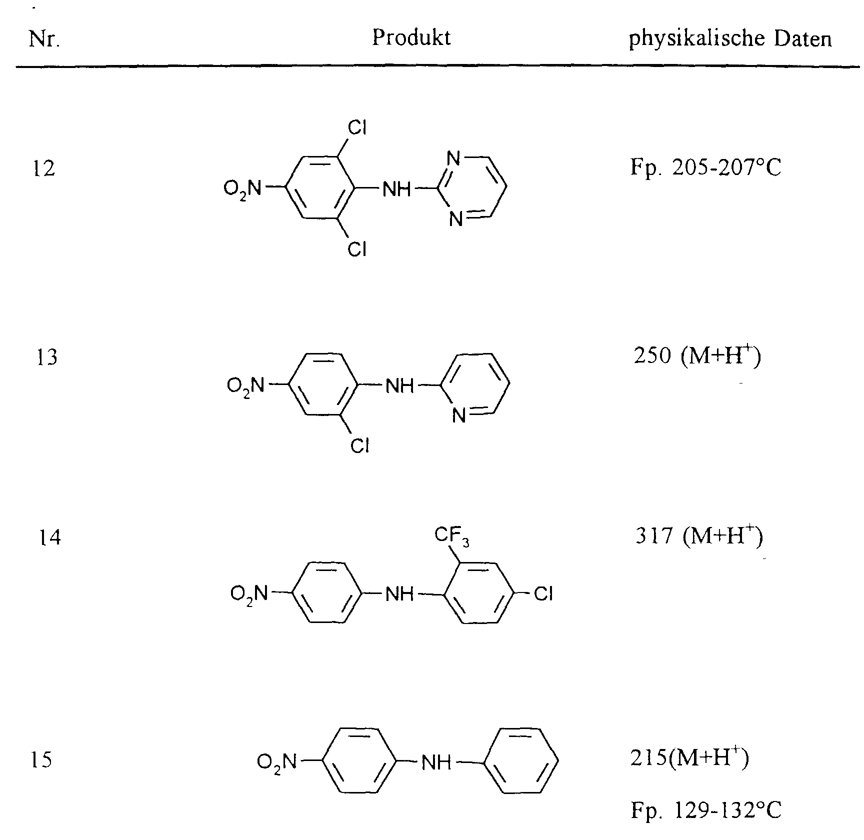

## Patentansprüche

1. Verfahren zur Herstellung von Verbindungen der Formel 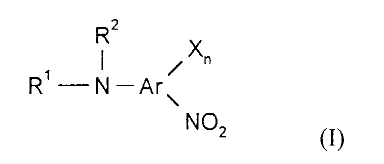 worin
Ar einen mono- oder polycyclischen aromatischen Rest mit 4 bis 16 C-Atomen, der auch 1 bis 2 Heteroatome aus der Reihe bestehend aus N, O und S enthalten kann,
R¹ einen mono- oder polycyclischen aromatischen Rest mit 5 bis 16 Ringgliedern, die aus Kohlenstoffatomen und gegebenenfalls bis zu 3 Heteroatomen aus der Reihe N, O und S bestehen, ausgewählt aus der Gruppe Pyrazol, Imidazol, 1,2,4-Triazol, Thiazol, Benzol, Naphthalin, Pyridin, Chinolin, Pyrimidin,
R² Wasserstoff, C₁-C₈-Alkyl, C₂-C₈-Alkenyl, C₃-C₇-Cycloalkyl, C₆-C₁₄-Aryl, wobei diese Substituenten 1- bis 3-fach durch Halogen, C₁-C₁-C₄-Alkyl, und/oder C₁-C₄-Alkoxy substituiert sein können,
X Halogen, Cyano, C₁-C₄-Alkyl, halogeniertes C₁-C₄-Alkyl, C₁-C₄-Alkoxy, halogeniertes C₁-C₄-Alkoxy, Trifluormethylmercapto, C₁-C₄-Alkylsulfonyl, Carboxyl oder Nitro,
n Null, 1, 2 oder 3 bedeuten,
wobei bei n > 1 die Substituenten X verschieden sein können,
wonach man Nitroaromaten der Formel 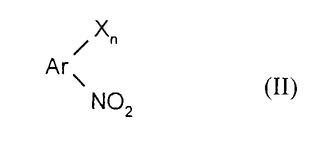 worin X, Ar und n die oben angegebenen Bedeutungen haben,
mit Aminen der Formel 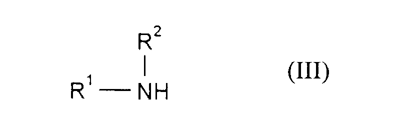 worin R1 und R² die oben angegebenen Bedeutungen besitzen,
in Gegenwart von Basen unter Einleiten von Sauerstoff in polaren Lösungsmitteln umsetzt.

2. Verfahren nach Anspruch 1, wonach die Base aus der Reihe Alkalihydroxide, Alkaliamide, Alkalialkoxide, Alkalihydride ausgewählt ist.

3. Verfahren nach Anspruch 1, wonach das Lösungsmittel ein polares aprotisches organisches Lösungsmittel oder Ammoniak ist.

4. Verfahren nach Anspruch 1, wonach die Reaktionstemperatur -35 bis 120°C beträgt.

5. Verfahren nach Anspruch 1, wonach man pro Mol des Nitroaromaten II 0,5 bis 10 Mol Amin III einsetzt.

## Claims

1. Process for the preparation of compounds of the formula 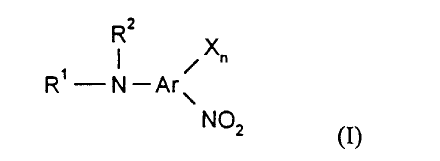 wherein
Ar denotes a mono- or polycyclic aromatic radical having 4 to 16 C atoms, which can also contain 1 to 2 heteroatoms from the series consisting of N, O and S,
R¹ denotes a mono- or polycyclic aromatic radical having 5 to 16 ring members, which consist of carbon atoms and optionally up to 3 heteroatoms from the series N, O and S, selected from the group pyrazole, imidazole, 1,2,4-triazole, thiazole, benzene, naphthalene, pyridine, quinoline, pyrimidine,
R² denotes hydrogen, C₁-C₈-alkyl, C₂-C₈-alkenyl, C₃-C₇-cycloalkyl or C₆-C₁₄-aryl, where these substituents can be substituted 1 to 3 times by halogen, C₁-C₄-alkyl and/or C₁-C₄-alkoxy,
X denotes halogen, cyano, C₁-C₄-alkyl, halogenated C₁-C₄-alkyl, C₁-C₄-alkoxy, halogenated C₁-C₄-alkoxy, trifluoromethylmercapto, C₁-C₄-alkylsulphonyl, carboxyl or nitro,
n denotes zero, 1, 2 or 3,
where if n > 1 the substituents X can be different,
according to which nitroaromatics of the formula 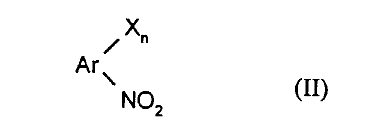 wherein X, Ar and n have the meanings indicated above,
are reacted with amines of the formula 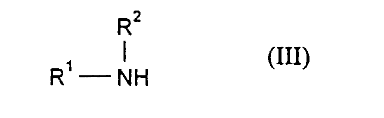 wherein R¹ and R² have the meanings indicated above,
in the presence of bases while introducing oxygen in polar solvents.

2. Process according to Claim 1, according to which the base is selected from the series alkali metal hydroxides, alkali metal amides, alkali metal alkoxides, alkali metal hydrides.

3. Process according to Claim 1, according to which the solvent is a polar aprotic organic solvent or ammonia.

4. Process according to Claim 1, according to which the reaction temperature is -35 to 120°C.

5. Process according to Claim 1, according to which 0.5 to 10 mol of amine III are employed per mole of the nitroaromatic II.

## Revendications

1. Procédé de préparation des composés de formule 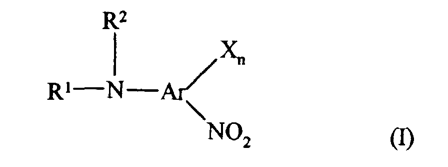 dans laquelle
Ar représente un radical aromatique mono- ou poly-cyclique en C₄-C₁₆, qui peut encore contenir 1 ou 2 hétéroatomes choisis parmi N, O et S,
R¹ représente un radical aromatique mono- ou poly-cyclique de 5 à 16 chaînons consistant en atomes de carbone et le cas échéant jusqu'à 3 hétéroatomes choisis parmi N, O et S, ce radical étant choisi parmi les radicaux du pyrazole, de l'imidazole, du 1,2,4-triazole, du thiazole, du benzène, du naphtalène, de la pyridine, de la quinoléine, de la pyrimidine,
R² représente l'hydrogène, un groupe alkyle en C₁-C₈, alcényle en C₂-C₈, cycloalkyle en C₃-C₇, aryle en C₆-C₁₄, lesquels peuvent eux-mêmes porter 1 à 3 substituants halogéno, alkyle en C₁-C₄ et/ou alcoxy en C₁-C₄,
X représente un halogène, un groupe cyano, alkyle en C₁-C₄, alkyle en C₁-C₄ halogéné, alcoxy en C₁-C₄, alcoxy en C₁-C₄ halogéné, trifluorométhylmercapto, alkylsulfonyle en C₁-C₄, carboxyle ou nitro,
n est égal à 0, 1, 2 ou 3,
les substituants X pouvant être différents lorsque n > 1,
dans lequel on fait réagir des dérivés nitroaromatiques de formule 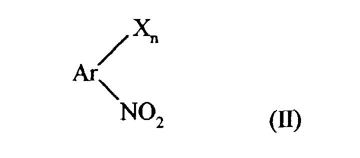 dans laquelle X, Ar et n ont les significations indiquées ci-dessus,
avec des amines de formule 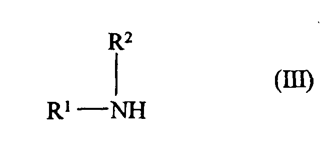 dans laquelle R¹ et R² ont les significations indiquées ci-dessus,
en présence de bases et sous injection d'oxygène dans des solvants polaires.

2. Procédé selon la revendication 1, dans lequel la base est choisie parmi les hydroxydes alcalins, les amidures alcalins, les alcoolates alcalins, les hydrures alcalins.

3. Procédé selon la revendication 1, dans lequel le solvant est un solvant organique aprotonique polaire ou l'ammoniac.

4. Procédé selon la revendication 1, dans lequel la température de réaction est comprise entre -35 et +120°C.

5. Procédé selon la revendication 1, dans lequel on utilise de 0,5 à 10 mol de l'amine III par mol du dérivé nitroaromatique II.
